# EUROPEAN PATENT SPECIFICATION

(11) **EP 1 425 005 B1**
(45) Date of publication and mention of the grant of the patent: **03.05.2006**
(21) Application number: 02767455.5
(22) Date of filing: 30.08.2002
(51) Int. Cl.: A61K 31/196, A61P 29/00

(54) **PHARMACEUTICAL COMPOSTION COMPRISING LUMIRACOXIB**
PHARMAZEUTISCHE ZUSAMMENSETZUNG ENTHALTEND LUMIRACOXIB
COMPOSITION PHARMACEUTIQUE COMPRENANT DU LUMIRACOXIB

(30) Priority: 31.08.2001 US 316389 P
(43) Date of publication of application: 09.06.2004
(73) Proprietor: Novartis AG, 4002 Basel (CH); Novartis Erfindungen Verwaltungsgesellschaft M.B.H., 1230 Vienna (AT)
(72) Inventor: KARNACHI, Anees Abdulquadar, Hillsborough, NJ 08844 (US); BATEMAN, Simon, David, Randolph, NJ 07869 (US)
(74) Representative: de Weerd, Petrus G.W.
(86) International application number: PCT/EP2002/009701
(87) International publication number: WO 2003/020261

(56) References cited:
- WO-A-02/20090
- WO-A-99/11605

## Description

This invention relates to compositions for the treatment of cyclooxygenase-2 mediated diseases and methods of treatment of cyclooxygenase-2 mediated diseases.

In particular, this invention relates to a composition for the treatment of cyclooxygenase-2 mediated diseases, the composition comprising about 65% active agent and being suitable for once a day administration, the composition comprising, as the active agent, a cyclooxygenase-2 inhibiting compound characterized by high potency for the inhibition of cyclooxygenase-2 and a high degree of specificity for inhibiting cyclooxygenase-2 in preference to cyclooxygenase-1. Such a compound is exemplified by 5-methyl-2-(2'-chloro-6'-fluoroanilino)phenylacetic acid.

Non-steroidal anti-inflammatory agents are normally administered 2 to 4 times daily. The relatively short half-life of most non-steroidal anti-inflammatory agents, such as aspirin, ibuprofen, naproxen, and diclofenac, means that once and even twice a day administration is not possible, unless the agent is formulated in a controlled or extended release formulation. The relatively large doses needed to achieve once a day treatment of conventional non-steroidal anti-inflammatory agents would also lead to side effects, if given in an immediate release formulation, so that there is a general understanding that once a day administration in an immediate release formulation is unlikely to be achievable.

Surprisingly a compound has been identified which can be employed on a once a day basis and which will not produce an unacceptable level of side effects on such a regimen, and in particular will not cause an unacceptable level of gastric side effects. WO 99/11605, published March 11, 1999 discloses 5-alkyl-2-arylaminophenylacetic acids and derivatives thereof as potent, selective inhibitors of cyclooxygenase-2. It has been found that 5-methyl-2-(2'-chloro-6'-fluoroanilino)phenylacetic acid possesses a surprising combination of attributes that make it possible to formulate and use the composition in a unexpected manner. This active agent, when administered once a day, in a single, immediate release tablet formulation in an amount of about 400 mg, where the active agent comprises about 65% by weight of the tablet, provides effective anti-inflammatory treatment over a 24 hour period, without the use of extended release pharmaceutical formulation excipients and technology, and in a tablet form which is of a size that is easy to swallow. Because the drug substance 5-methyl-2-(2'-chloro-6'-fluoroanilino)phenylacetic acid and its pharmaceutically acceptable salts have been found to exhibit poor compressibility, it was surprising and unexpected that a tablet dosage form with between a 60% and a 70% drug load could be achieved.

### SUMMARY OF THE INVENTION

In a first aspect, the invention is directed to a composition for treating a cyclooxygenase-2 dependent disorder or condition, the composition comprising:

an immediate release tablet comprising about 400 mg of 5-methyl-2-(2'-chloro-6'-fluoroanilino)phenylacetic acid or a pharmaceutically acceptable salt thereof, where the tablet comprises between 60% and 70% of 5-methyl-2-(2'-chloro-6'-fluoroanilino)phenylacetic acid or a pharmaceutically acceptable salt thereof by weight.

Disclosed is also a composition for treating a cyclooxygenase-2 dependent disorder or condition, the composition comprising:

(a) an immediate release tablet comprising about 400 mg of 5-methyl-2-(2'-chloro-6'-fluoroanilino)phenylacetic acid or a pharmaceutically acceptable salt thereof, where the tablet comprises between 60% and 70% of 5-methyl-2-(2'-chloro-6'-fluoroanilino)phenylacetic acid or a pharmaceutically acceptable salt thereof by weight and

(b) printed instructions directing that the one or more immediate release pharmaceutical compositions comprising 5-methyl-2-(2'-chloro-6'-fluoroanilino)phenylacetic acid be administered orally once a day.

### DETAILED DESCRIPTION OF THE INVENTION

The present invention is directed to a composition for the treatment of cyclooxygenase-2 mediated diseases, the composition comprising a cyclooxygenase-2 inhibiting compound characterized by:

(a) high potency for the inhibition of cyclooxygenase-2 as measured by the ability of a single therapeutic dose of the compound to provide relief from osteoarthritis pain;

(b) a half-life 3 to 6 hours; and

(c) a high degree of specificity for inhibiting cyclooxygenase-2 in preference to cyclooxygenase-1 as measured by *in vitro* cellular assays as described in WO 99/11605.

One such compound is 5-methyl-2-(2'-chloro-6'-fluoroanilino)phenylacetic acid. 5-methyl-2-(2'-chloro-6'-fluoroanilino)phenylacetic acid, the utility of which and methods of synthesis of which are disclosed in WO 99/11605.

Thus, the present invention is directed to a an immediate release tablet comprising about 400 mg of 5-methyl-2-(2'-chloro-6'-fluoroanilino)phenylacetic acid or a pharmaceutically acceptable salt thereof, where the tablet comprises between 60% and 70% of 5-methyl-2-(2'-chloro-6'-fluoroanilino)phenylacetic acid or a pharmaceutically acceptable salt thereof by weight. In a particular embodiment, the immediate release tablet comprises about 65% of 5-methyl-2-(2'-chloro-6-fluoroanilino)phenylacetic acid or a pharmaceutically acceptable salt thereof by weight.

The immediate release composition(s) does not comprise sufficient water-insoluble or polymeric components to impart extended release characteristics to the composition.

As discussed in WO 99/11605, a genus of compounds, including 5-methyl-2-(2'-chloro-6'-fluoroanilino)phenylacetic acid, is useful for the relief of pain, fever and inflammation of a variety of conditions including rheumatic fever, symptoms associated with influenza or other viral infections, common cold, low back and neck pain, dysmenorrhea, headache, including migraine headache, toothache, sprains and strains, myositis, neuralgia, synovitis, arthritis, including osteoarthritis and rheumatoid arthritis, degenerative joint diseases, gout and ankylosing spondylitis, bursitis, bums, and injuries following surgical and dental procedures.

By virtue of its high cyclooxygenase-2 (COX-2) inhibitory activity and/or its selectivity for inhibiting COX-2 over cyclooxygenase-1 (COX-1), 5-methyl-2-(2'-chloro-6'-fluoroanilino)phenylacetic acid is also useful as an alternative to conventional non-steroidal anti-inflammatory drugs (NSAID'S) particularly where such NSAIDS may be contra-indicated such as in patients with peptic ulcers, gastritis, regional enteritis, ulcerative colitis, diverticulitis or with a recurrent history of gastrointestinal lesions; GI bleeding, coagulation disorders including anemia such as hypoprothrombinemia, haemophilia or other bleeding problems (including those relating to reduced or impaired platelet function); kidney disease (e.g. impaired renal function); those prior to surgery or taking anticoagulants; and those susceptible to NSAID induced asthma.

The immediate release pharmaceutical compositions of the invention, and which are useful in the kits and methods of the invention are "immediate release" tablet dosage forms. The tablets of the invention have neither the pharmacokinetic nor physical characteristics of extended release pharmaceutical dosage forms. Thus, a tablet of the invention will disintegrate or dissolve rapidly, preferably within one hour of administration, and administration of a tablet of the invention will result in a rapid rise in the blood plasma concentration of 5-methyl-2-(2'-chloro-6'-fluoroanilino)phenylacetic acid. Preferably, the blood plasma concentration of 5-methyl-2-(2'-chloro-6'-fluoroanilino)phenylacetic acid will reach a maximum within two to six hours after oral administration and will then fall relatively rapidly due to the relatively short (3 to 6 hour) half life of 5-methyl-2-(2'-chloro-6'-fluoroanilino)phenylacetic acid.

Non-immediate release drug formulations, which are not within the scope of the present invention or used therein, include, *inter alia,* delayed release and sustained release formulations. Sustained release formulations may be further subdivided into prolonged release and controlled release formulations. Delayed release systems are those that use repetitive, intermittent dosing of a drug from one or more immediate-release units incorporated into a single dosage form. Examples of delayed release formulations include repeat-action tablets and capsules, and enteric-coated tablets where timed release is achieved by a barrier coating. Delayed release formulations do not produce or maintain uniform blood plasma concentrations of drug, but rather produce intermittent peaks and troughs in the blood plasma concentration of a drug, which are both desirably within the therapeutic range for the drug.

Sustained release drug formulations include drug formulations that achieve slow release of a drug over an extended period of time. If a sustained release formulation can maintain a constant drug concentration in the blood plasma, it is referred to herein as a "controlled release" formulation. If it does not maintain a constant concentration of drug in the blood plasma, but maintains the concentration of the drug in the therapeutic range for a longer period of time than would be achievable with an immediate release formulation, it is referred to herein as a "prolonged release" formulation. Thus, controlled release formulations maintain a relatively constant, peak blood plasma concentration of drug over an extended period of time, typically twelve to twenty four hours; the compositions of the present invention do not.

Typically, sustained release oral dosage formulations are based on a diffusion system, a dissolution system, and osmotic system, or an ion-exchange system.

In diffusion systems, the release rate of the drug is determined by its diffusion through a water-insoluble polymer. There are two types of diffusion devices: reservoir devices, in which a core of drug is surrounded by a polymeric membrane, and matrix devices, in which dissolved or dispersed drug is distributed uniformly throughout an inert polymeric matrix. Typical methods used to make reservoir-type devices include microencapsulation of drug particles and press-coating of whole tablets or particles. Generally, particles coated by microencapsulation form a system where the drug is contained in the coating film as well as in the core of the microcapsule. Some materials typically used as the water-insoluble coating, alone or in combination, are hardened gelatin, methyl or ethylcelluloses, polyhydroxymethacrylate, hydroxypropylcellulose, polyvinylacetate, and waxes.

Matrix devices are typically made by mixing drug with matrix material and then compressing the mixture into tablets. When using wax matrices, drug is generally dispersed in molten wax, which is then congealed, granulated, and compressed into cores. Matrix systems typically have a priming dose of drug coated onto the drug-matrix core. The major types of materials used in the preparation of matrix devices are insoluble plastics, hydrophilic polymers and fatty compounds. Plastic matrices include methyl acrylate-methyl methacrylate, polyvinyl chloride and polyethylene. Hydrophilic polymers include methylcellulose, hydroxypropylmethylcellulose and sodium carboxymethylcellulose. Fatty compounds include waxes such as carnauba wax and glyceryl tristearate.

Most dissolution type sustained release formulations are either encapsulated dissolution systems or matrix dissolution systems. Encapsulated dissolution formulations can be prepared either by coating particles or granules of drug with varying thicknesses of slowly soluble polymers or by microencapsulation. A common method of microencapsulation is coacervation, which involves the addition of a hydrophilic substance to a colloidal dispersion. The hydrophilic substance, which coats the suspended particles, can be selected from a wide variety of natural and synthetic polymers including shellacs, waxes, starches, cellulose acetate phthalate (or butyrate) or polyvinylpyrrolidone. Once the coating material dissolves, all of the drug inside the microcapsule is available immediately for dissolution and absorption, allowing drug release to be controlled by adjusting the thickness and dissolution rate of the coat. If three or four coating thicknesses are used in the microcapsules that comprise a formulation, drugs will be released at different, predetermined times to give a delayed-release, pulsatile effect. If a spectrum of thicknesses is employed, a more constant blood concentration of the drug can be achieved. Encapsulated particles can be compressed into tablets or placed into capsules.

Matrix dissolution sustained release formulations are prepared by preparing particles comprising drug and slowly soluble polymer particles. Such particles can be prepared by congealing drug with a polymer or wax and spray-congealing the particles or by cooling the drug-coating mixture and screening it. Alternatively, an aqueous dispersion method can be used, where a drug-polymer mixture is sprayed or placed in water and the resulting particles are collected. The drug-polymer particles are then compressed into tablets.

Formulations that rely on osmotic gradients have also been used to provide sustained release of drug. Typically, such formulations involve a membrane, permeable to water but not drug, that surrounds a core of drug. The membrane has a small delivery aperture. Water flows through the semipermeable membrane, dissolves drug, which is then pumped out of the formulation through the delivery aperture. Materials that can be used as a semipermeable membrane are polyvinyl alcohol, polyurethane, cellulose acetate, ethylcellulose, and polyvinyl chloride.

The immediate release tablets of the invention are intended for oral use.

5-methyl-2-(2'-chloro-6'-fluoroanilino)phenylacetic acid has surprisingly been found to possess a duration of action in humans of sufficient length that a single oral dose of about 400 mg of drug per day in an immediate-release tablet that comprises about 65% by weight of 5-methyl-2-(2'-chloro-6'-fluoroanilino)phenylacetic acid will provide effective, safe anti-inflammatory treatment over a 24 hour period. Without being bound by theory, clinical data suggest that the effect of 5-methyl-2-(2'-chloro-6'-fluoroanilino)phenylacetic acid on pain is not directly related to plasma concentration, but rather may be governed by the concentrations of drug in an effect compartment. Such an agent is particularly useful in the treatment of chronic indications, such as rheumatoid arthritis and osteoarthritis as well as Alzheimer's Disease and prophylaxis of colon cancer, where the drug needs to be taken every day for the duration of a subject's life, because compliance is much easier with once a day dosing.

400 mg of 5-methyl-2-(2'-chloro-6'-fluoroanilino)phenylacetic acid or a salt thereof has been found to be an effective analgesic dose. A single, 400 mg tablet that is easy to swallow facilitates compliance with a once-a-day, 400 mg dose treatment regimen.

A useful 400 mg tablet will have have a friability of less than or equal to 1%, such as between 0.1% and 0.6% or between 0.4% and 0.6%; a hardness between 130N and 260N; a disintegration time of less than about 10 minutes; will release at least about 70% of drug in 60 minutes at 37°C in 1000 mL of pH 8 phosphate buffer with 0.1% Tween 80; and will require an ejection force from the tablet press in which it is formed of less than about 1000 N.

The resistance of the tablet to chipping, abrasion, or breakage under conditions of storage, handling, transportation and handling before usage depends on its hardness. The apparatus used to measure the tablet hardness or crushing strength is the Schleuuniger apparatus. Hardness determinations are made throughout the tablet runs to determine the need for pressure adjustments on the tableting machine. If the tablet is too hard, it may not disintegrate in the required period of time or meet the dissolution specification; if it is too soft, it will not withstand the handling during subsequent processing such as coating or packaging and shipping operations. The hardness specifications for the tablets of the invention are a target of 190 N, with a range of 170 to 220 N for an average of 20 cores, with an individual range of 130 to 260 N.

As used herein, disintegration time is the amount of time for a tablet to reach a state in which any residue of the tablet, except fragments of insoluble coating or capsule shell, remaining on the screen of the test apparatus is a soft mass having no palpably firm core.

The test apparatus consists of a basket-rack assembly holding six plastic tubes open at the top and bottom and the bottom of the tubes is covered with 10 mesh screen, a 1000 mℓ low-form beaker for the immersion fluid, a thermostatic arrangement for heating the fluid between 35 and 39°C, and a device for raising and lowering the basket in the immersion fluid at a constant frequency rate between 29 and 32 cycles per minute through a distance of not less than 5.3 cm and not more than 5.7 cm. The volume of the fluid in the vessel is such that at the highest point of the upward stroke the wire mesh remains at least 2.5 cm below the surface of the fluid and descends to not less tan 2.5 cm from the bottom of the vessel on the downward stroke. The time required for the upward stroke is equal to the time required for the downward stroke, and the change in stroke direction is a smooth transition, rather than an abrupt reversal of motion. The basket assembly moves vertically along its axis. There is no appreciable horizontal motion or movement of the axis from the vertical. To test for disintegration, a tablet is placed in each of the six tubes of the basket and the apparatus is operated using water maintained at 37°C as the immersion fluid. The end point of the test is indicated when any residue remaining is a soft mass having no palpably soft core.

The tablets of the invention have drug release properties such that in a dissolution medium consisting of 1000 ml of pH 8.0 phosphate buffer with 0.1% Tween 80, being stirred at 50 rpm, at least 70% of the drug dissolves in 60 minutes. Most preferably, 75% of the drug dissolves in 15 minutes.

### Example 1

To develop a high drug loading 400 mg active agent composition, high shear granulation and spray granulation processes are evaluated in parallel. The drug substance is granulated with povidone solution. Povidone concentrations of 4 % (96% drug loading) and 8 % (92% drug loading) are used. The granulations are dried and milled through a # 20 mesh (0.84 mm). Portions of granules that are retained on the 60 mesh (0.25 mm) and 80 mesh (0.175 mm) are mixed and are compressed on a carver press. Round 11 mm flat face beveled edge tooling is used and compression forces ranging from 2 to 10 KN (Kilonewtons) are applied to generate a compression profile. Increase in the tensile strength is evaluated by determining the hardness of the compacts upon crushing. Increasing the force from 2 KN to 10 KN resulted in harder compacts with tensile strengths ranging from 78 N (Newtons) to 137 N. Evaluations are performed in duplicate. The tensile strength of compacts granulated with 8% povidone is slightly higher than compacts granulated with 4% povidone. However, with both concentrations there is no substantial difference in hardness values observed between granules that are made with either spray granulation or the high shear granulation.

Further experiments are conducted by processing these granulations into feasible formulations and compressibility is evaluated. Since it is desired to make as small tablet as possible three different drug loading levels are evaluated. Drug loading using 85%, 75%, and 65% active are studied. This is performed for both the high shear and spray granulation processes. The drug substance is granulated with a povidone solution. The granulation is then dried and milled. The following components are added extragranularly to the milled granules: microcrystalline cellulose (PH 102), croscarmellose sodium, and magnesium stearate. Blends are compressed on a Beta press using appropriate tooling size and compressibility is studied.

The higher drug load at 85% active is evaluated first. Compression profiles generated demonstrate that the high shear granulation process results in harder tablets with increasing applied forces. Compression forces greater than 12 kilonewtons (KN) cause lamination of the 85% active tablets. In comparison, lamination is observed at forces higher than 9 KN for the spray granulation batch. Both of the formulations with 85% drug loading result in high friability values in excess of 2% after 200 drops. Friability is a measure of the brittleness of tablets, and is measured by weighing tablets before and after they have been subjected to "dropping", and dividing the difference between tablet weight before dropping and the tablet weight after dropping by the tablet weight before dropping and multiplying by 100. Friability is measured using a friabilitor, which is a rotating drum that, every revolution, drops tablets enclosed therein a distance of 6 inches. Typically, about 20 tablets are used for each friability test. Acceptable friability is defined as less than 1%. Accordingly, compressibility is not satisfactory at 85% drug loading.

Drug loading at 75% and 65% active is evaluated. The high shear batch has better compressibility than the spray granulation batch at the forces applied. Tablets laminate upon crushing at 16 KN force for the high shear process as compared to lamination at 12 KN force for the spray granulation batch. Although friability values are still unacceptably high after 500 drops, data from these experiments suggest that 65% drug loading can yield tablets with satisfactory compression properties. High shear granulation with 65% drug loading is selected for further formulation development.

Evaluation of 400 mg tablet formulations was based on a unit weight of cores of 615 mg containing 400 mg of drug substance, which is 65.04% drug load, with povidone as a binder. The disintegrant (croscarmellose sodium) is split equally into intragranular and extragranular portions. The extragranular portion disintegrates tablets into granules and the intragranular portion reduces granules to even finer particles, facilitating dissolution and release. The filler, microcrystalline cellulose (PH 102) is added extragranularly as is magnesium stearate, which is used as a lubricant. Three formulation factors are studied at three different levels and five responses evaluated. These are shown in Table 1.

**Table 1 Formulation variables and responses.**

| **Factors** | **Levels** | **Responses** |
|---|---|---|
| Binder (Polyvinylpyrrolidone, Povidone K30) | 4%, 6%, 8% | Compressibility |
| Disintegrant (croscarmellose sodium, Ac-Di-Sol) | 2%, 4%, 6% | Ejection force |
| Lubricant (Magnesium Stearate) | 0.5%,1%, 1.5% | Friability (500 drops) Disintegration time Dissolution rate |

The tablets are formulated by first dissolving the polyvinylpyrrolidone binder in water. The drug substance and croscarmellose sodium are added to the Gral mixer and mixed. This mixture is granulated using the polyvinylpyrrolidone solution. The resulting wet granulation is dried in a fluid bed dryer, and is screened using an oscillating 18 mesh screen. Microcrystalline cellulose (Avicel PH-102, NF) and croscarmellose sodium are screened using an 18 mesh screen blended with the screened, dried granulation of polyvinylpyrrolidone, drug substance, and croscarmellose sodium The resulting mixture is then blended with magnesium stearate that has been screened through an 18 mesh screen. The resulting final blend is then compressed on a tablet press. An eight run experimental design is generated with two replicate runs (run 7 and 8). Table 2 shows the experimental runs.

**Table 2 Experimental runs**

| **Experiment #** | **Binder (%)** | **Disintegrant (%)** | **Lubricant (%)** |
|---|---|---|---|
| 1 | 4 | 6 | 1 |
| 2 | 8 | 2 | 1 |
| 3 | 7.5 | 2 | 1.5 |
| 4 | 4.5 | 6 | 0.5 |
| 5 | 8 | 2.5 | 0.5 |
| 6 | 4 | 5.5 | 1.5 |
| 7 | 6 | 4 | 1 |
| 8 | 6 | 4 | 1 |

The following process conditions are kept similar for all the eight experiments. These include:

Equipment: 10L Gral mixer, GPCG-1 dryer, Frewitt mill, 4 Qt V-blender, Beta Press

Water amount: 18.5% of the sum of drug substance, croscarmellose sodium and povidone weight.

Binder addition rate: 3 minutes 30 seconds for all the batches

Granulation time: between 30 seconds to 5 minutes

Drying temperature: 50°C

Residual moisture: 0.5 to 2% (%LOD)

Mesh size: US standard #18 (1.0 mm)

Tablet tooling: 17 X 6.7 mm Ovaloid with NVTRD debossed on one side and 984 on the other side.

Tablet press: Beta press with gravity feed frame

Tablet press speed: 75-80 RPM

Tablets are compressed at various forces to generate compression profiles. The compression and ejection forces are monitored during compression using an instrumented tablet press. Friability, disintegration time and dissolution of the cores are also evaluated. Table 3 shows the data at 13-16 KN force for the eight experiments.

**Table 3 Tablet physical characterization data for the experimental runs**

| **Compression force (KN)** | **Hardness (N)** | **Ejection force (N)** | **DT mins:secs** | **Dissolution at 15 mins (%)** | **Dissolution at 60 mins (%)** | **Friability (%)** |
|---|---|---|---|---|---|---|
| 14.9 | 195 | 590 | 1:48-2:42 | 81.9 | 93.4 | 1.26 |
| 14.8 | 166 | 546 | 9:30-10:18 | 73.7 | 85.1 | 130 |
| 14.5 | 177 | 511 | 8:32-10:13 | 73.1 | 83.9 | 1.03 |
| 15.2 | 187 | 648 | 3:05-3:50 | 81.8 | 89.5 | 0.90 |
| 14.6 | 188 | 583 | 8:45-9:20 | 67.5 | 87.0 | 0.40 |
| 13.9 | 167 | 542 | 2:07-2:28 | 74.6 | 90.0 | 2.45 |
| 13.9 | 186 | 526 | 5:15-5:58 | 75.6 | 90.1 | 1.02 |
| 13.6 | 175 | 516 | 4:47-5:32 | 78.5 | 87.6 | 0.83 |

Friability is measured after 500 drops, and the acceptable dissolution standard is 70% dissolved in 60 minutes. It is observed that binder and disintegrant had a significant effect on disintegration time, dissolution, hardness and ejection forces. Despite the different levels of binder and disintegrant, all batches pass the dissolution test (Q point of 70% drug released in 60 minutes). Lubricant levels had a significant effect on friability after 500 drops, but had no significant effect on ejection forces. For further optimization, friability is optimized as this response is considered most critical for successful scale-up and developing a robust coated tablet. Friability of less than 1 % (preferably around 0.4 - 0.6%) after 500 drops is targeted for optimization. Table 4 lists the constraints on acceptable tablet properties.

**Table 4 Constraints on tablet properties**

| **Responses** | **Constraints** |
|---|---|
| Friability | Less than 1% |
| Hardness | Greater than 147N |
| Disintegration time | Less than 9 minutes |
| Dissolution (after 15 minutes) | 75 % drug released in 15 minutes |
| Ejection force | Less than 1000 N |

**Table 5 Optimized factor levels and predicted responses**

| **Optimized factor levels** | **Predicted responses** |
|---|---|
| Binder 6.55% | Friability 0.5% |
| Disintegrant 4.26% | Hardness 193 N |
| Lubricant 0.42% | Disintegration time 6 minutes 36 seconds Dissolution after 15 minutes 75% Ejection force 634 N |

An optimized formulation is manufactured at with 6.55% binder, 4.26% disintegrant, and 0.42% lubricant levels, which yielded the properties set out in Table 6 below.

**Table 6 Observed and predicted values of the properties for optimized formulation at 14 KN compression force**

| **Properties** | **Observed** |
|---|---|
| Friability | 0.72 % |
| Hardness | 179 N (170 - 190) |
| Ejection force | 688 N |
| Disintegration time (mins:secs) Dissolution after 15 minutes | 6:54 - 7:28 75.5 % |

The optimized formulation is further stressed for friability by increasing the lubricant concentration to 0.75%. Hardness, dissolution and disintegration times are not affected by increasing the lubricant concentration. However, friability is higher at 14 KN compression force. Increasing the compression force to 16 KN results in acceptable friability (0.47%).

The optimized formulation is as set out in Table 7, with information about as percentage w/w and mg/dose. This is a large batch with a batch size of 50,000 tablets manufactured in a 75L Gral mixer. Based on data from development batches, minor changes in excipient concentrations will not affect the overall product attributes.

**Table 7 Optimized formulation composition**

| **% w/w** | **Ingredient** | **Mg/dose** |
|---|---|---|
| | Granulation | |
| 65.04 | Drug substance | 400.00 |
| 2.15 | Croscarmellose sodium, NF (Ac-Di-Sol) | 13.20 |
| 6.60 | Povidone K30, USP | 40.60 |
| 18.12 | Purified water, USP* | Qs |

| | Blending | |
|---|---|---|
| 23.56 | Microcrystalline Cellulose, NF (Avicel PH 102) | 144.90 |
| 2.15 | Croscarmellose sodium, NF (Ac-Di-Sol) | 13.20 |
| 0.50 | Magnesium Stearate, NF (vegetable source) | 3.10 |

| | Film Coating | |
|---|---|---|
| 84.44 | Opadry, Global White 00F18296 | 15.20 |
| 13.90 | Opadry, Global Red 00F15613 | 2.50 |
| 1.51 | Opadry, Global Black 00F17713 | 0.3 |
| | Purified Water, USP* | Qs |
| | Film Coated Tablet Weight | 633.00 |

| | | |
|---|---|---|
| *Does not appear in final product. Percentage of water added used for granulation based on the dry weight of drug substance and croscarmellose sodium. | | |

The batch is granulated as described above for development batches. The granulation is dried to residual moisture in the range of 1.5-2.5% LOD (loss on drying). The formulation process is the same as for the development batches as described above, except for the additional step of coating with Opadry in a coating pan. The coating powders (Opadry) are mixed with purified water to make a 15 % w/w coating suspension. The tablets are film coated with the coating suspension in a coating pan using 60°C to 75°C inlet air temperature. Based on friability data, a target hardness of 190N (170-210N) which corresponds to 18 KN force (16 to20 KN) is used to compress the remainder of the batch, resulting in acceptable friability (less than 0.5%) and the disintegration times of less than 5 minutes. The ejection force is approximately 800 N throughout the compression run. This demonstrates that the blend is lubricated adequately. No picking/sticking is observed on the punch surfaces after 225 minutes of compression run time. Thus, a smaller size tablet with high drug loading (65%) is achieved using a high shear granulation process, using 17 X 6.7 mm ovaloid tooling to get tablets with acceptable hardness, friability, disintegration time and dissolution characteristics.

In addition, the tablet formulations may contain 5-methyl-2-(2'-chloro-6'-fluoroanilino)benzyl alcohol and/or 5-methyl-2-(2'-chloro-6'-fluoroanilino)benzoic acid in an amount between about 0.01 and 2% by weight, more specifically between about 0.1 and 1%. Thus, in a further embodiment the present invention is directed to a pharmaceutical composition comprising an effective amount of 5-methyl-2-(2'-chloro-6'-fluoroanilino)phenylacetic acid and between 0.01 and 2% by weight of 5-methyl-2-(2'-chloro-6'-fluoroanilino)benzyl alcohol.

## Claims

1. A composition for treating a cyclooxygenase-2 dependent disorder or condition comprising:
an immediate release tablet comprising about 400 mg of 5-methyl-2-(2'-chloro-6'-fluoroanilino)phenylacetic acid or a pharmaceutically acceptable salt thereof, wherein the tablet comprises between 60% and 70% by weight of 5-methyl-2-(2'-chloro-6'-fluoroanilino)phenylacetic acid or a pharmaceutically acceptable salt thereof, wherein said immediate release tablet does not comprise sufficient water-insoluble or polymeric components to impart extended release characteristics to said composition.

2. A composition according to claim 1, wherein said composition comprises about 65% by weight of 5-methyl-2-(2'-chloro-6'-fluoroanilino)phenylacetic acid or a pharmaceutically acceptable salt thereof.

3. The composition of claim 2, wherein said composition comprises polyvinylpyrrolidone, croscarmellose sodium, magnesium stearate, and microcrystalline cellulose.

4. The composition of claim 3, wherein said composition comprises, by weight, about 6.6% polyvinylpyrrolidone, about 4.3% croscarmellose sodium, about 0.5% magnesium stearate, and about 23.56% microcrystalline cellulose.

5. The composition of claim 4, wherein said tablet comprises about 65% by weight of 5-methyl-2-(2'-chloro-6'-fluoroanilino)phenylacetic acid or a pharmaceutically acceptable salt thereof.

6. The composition of any one of the preceding claims, wherein said tablet has a friability less than 1%.

7. The composition of any one of the preceding claims, wherein said tablet has a hardness between 130 and 260 N.

8. The composition of any one of the preceding claims, wherein said tablet has a disintegration time of less than about 10 minutes.

9. The composition of any one of the preceding claims, wherein said tablet will release drug such that at least about 70% of said drug dissolves in 1000 mℓ of pH 8 phosphate buffer with 0.1% Tween 80 in 60 minutes at 37°C.

## Patentansprüche

1. Zusammensetzung zur Behandlung von Cyclooxygenase-2 abhängiger Störung oder eines solchen Zustands, die umfasst
eine unmittelbar freisetzende Tablette, die etwa 400 mg an 5-Methyl-2-(2'-chlor-6'-fluoranilino)phenylessigsäure oder einem pharmazeutisch annehmbaren Salz hiervon enthält, worin die Tablette zwischen 60 und 70 Gewichtsprozent an 5-Methyl-2-(2'-chlor-6'-fluoranilino)phenylessigsäure oder einem pharmazeutisch annehmbaren Salz hiervon enthält, worin diese unmittelbar freisetzende Tablette keine ausreichenden wasserunlöslichen oder polymeren Komponenten aufweist, um der Zusammensetzung verlängert freisetzende Charakteristiken zu verleihen.

2. Zusammensetzung nach Anspruch 1, worin die Zusammensetzung etwa 65 Gewichtsprozent an 5-Methyl-2-(2'-chlor-6'-fluoranilino)phenylessigsäure oder einem pharmazeutisch annehmbaren Salz hiervon enthält.

3. Zusammensetzung nach Anspruch 2, worin die Zusammensetzung Polyvinylpyrrolidon, Croscarmellose-Natrium, Magnesiumstearat und mikrokristalline Cellulose umfasst.

4. Zusammensetzung nach Anspruch 3, worin die Zusammensetzung etwa 6,6 % Polyvinylpyrrolidon, etwa 4,3 % Croscarmellose-Natrium, etwa 0,5 % Magnesiumstearat und etwa 23,56 % mikrokristalline Cellulose bezogen auf das Gewicht enthält.

5. Zusammensetzung nach Anspruch 4, worin die Tablette etwa 65 Gewichtsprozent an 5-Methyl-2-(2'-chlor-6'-fluoranilino)phenylessigsäure oder einem pharmazeutisch annehmbaren Salz hiervon umfasst.

6. Zusammensetzung nach einem der vorangehenden Ansprüche, worin die Tablette eine Bröckeligkeit von weniger als 1 % aufweist.

7. Zusammensetzung nach einem der vorangehenden Ansprüche, worin die Tablette eine Härte zwischen 130 und 260 N aufweist.

8. Zusammensetzung nach einem der vorangehenden Ansprüche, worin die Tablette eine Zerfallszeit von weniger als etwa 10 Minuten aufweist.

9. Zusammensetzung nach einem der vorangehenden Ansprüche, worin die Tablette das Arzneimittel so freisetzt, dass zumindest 70 % des Arzneimittels in 1000 ml an pH 8 Phosphatpuffer mit 0,1 % Tween 80 in 60 Minuten bei 37°C freigesetzt werden.

## Revendications

1. Composition pour traiter une affection ou un état sous la dépendance de la cyclooxygénase-2, comprenant :
un comprimé à libération immédiate comprenant environ 400 mg d'acide 5-méthyl-2-(2'-chloro-6'-fluoroanilino)phénylacétique, ou de l'un de ses sels pharmaceutiquement acceptables, dans laquelle le comprimé comprend 60 % à 70 % en poids d'acide 5-méthyl-2-(2'-chloro-6'-fluoroanilino)phénylacétique, ou de l'un de ses sels pharmaceutiquement acceptable, ledit comprimé à libération immédiate ne comprenant pas une quantité suffisante de composants insolubles dans l'eau ou polymériques pour conférer des caractéristiques de libération prolongée à ladite composition.

2. Composition selon la revendication 1, ladite composition comprenant environ 65 % en poids d'acide 5-méthyl-2-(2'-chloro-6'-fluoro-anilino)phénylacétique, ou de l'un de ses sels pharmaceutiquement acceptables.

3. Composition selon la revendication 2, ladite composition comprenant de la polyvinylpyrrolidone, de la croscarmellose sodique, du stéarate de magnésium et de la cellulose microcristalline.

4. Composition selon la revendication 3, ladite composition comprenant, en poids, environ 6,6 % de polyvinylpyrrolidone, environ 4,3 % de croscarmellose sodique, environ 0,5 % de stéarate de magnésium, et environ 23,56 % de cellulose microcristalline.

5. Composition selon la revendication 4, dans laquelle ledit comprimé comprend environ 65 % en poids d'acide 5-méthyl-2-(2'-chloro-6'-fluoroanilino)phénylacétique, ou de l'un de ses sels pharmaceutiquement acceptables.

6. Composition selon l'une quelconque des revendications précédentes, dans laquelle ledit comprimé présente une friabilité inférieure à 1 %:

7. Composition selon l'une quelconque des revendications précédentes, dans laquelle ledit comprimé présente une dureté de 130 à 260 N.

8. Composition selon l'une quelconque des revendications précédentes, dans laquelle ledit comprimé présente un temps de désintégration inférieur à environ 10 minutes.

9. Composition selon l'une quelconque des revendications précédentes, dans laquelle ledit comprimé libérera le médicament de telle sorte qu'environ 70 % dudit médicament se dissolvent dans 1000 ml de tampon phosphaté à pH 8 contenant 0,1 % de Tween 80 en 60 minutes à 37°C.
